# FASCICULE DE BREVET EUROPEEN

(11) **EP 3 303 923 B1**
(45) Date de publication et mention de la délivrance du brevet: **12.05.2021**
(21) Numéro de dépôt: 16727432.3
(22) Date de dépôt: 31.05.2016
(51) Int. Cl.: F23G 7/06, H05B 3/40, F23G 5/24, F23C 3/00, F23C 99/00, F23G 5/10

(54) **DISPOSITIF DE PRODUCTION DE GAZ METHANE ET UTILISATION D'UN TEL DISPOSITIF**
METHAN GAS HERSTELLVORRICHTUNG UND NUTZUNGSANLAGE
METHANE GAS PRODUCTION DEVICE AND USAGE

(30) Priorité: 05.06.2015 FR 1555148; 15.09.2015 FR 1558609
(43) Date de publication de la demande: 11.04.2018
(73) Titulaire: E.T.I.A. - EVALUATION TECHNOLOGIQUE, INGENIERIE ET APPLICATIONS, 60201 Compiègne cedex (FR)
(72) Inventeur: LEPEZ, Olivier, 60260 Lamorlaye (FR); SAJET, Philippe, 60610 Lacroix-Saint-Ouen (FR)
(74) Mandataire: Decorchemont, Audrey Véronique Christèle
(86) Numéro de dépôt international: PCT/EP2016/062310
(87) Numéro de publication internationale: WO 2016/193273

(56) Documents cités:
- EP-A1- 2 690 162
- WO-A1-2013/053380
- FR-A1- 2 983 203
- FR-A1- 3 007 120
- US-A1- 2002 117 388
- US-A1- 2009 007 484
- US-A1- 2011 171 063
- US-A1- 2015 001 061

## Description

L'invention concerne un dispositif de production d'un gaz méthane c'est-à-dire un gaz ayant comme principal composant du méthane. L'invention concerne également l'utilisation d'un tel dispositif pour la valorisation d'un produit de type matière CSR (composés solides de récupération) ou matière polymérique.

### ARRIERE PLAN TECHNOLOGIQUE DE L'INVENTION

Bien que prisé pour ses propriétés énergétiques et environnementales, le gaz naturel présente l'inconvénient d'être une denrée non renouvelable. Or les réserves mondiales s'épuisent rapidement.

Le gaz naturel étant composé en moyenne à 95 % de méthane, il a été envisagé de profiter du phénomène naturel de méthanisation des déchets organiques produisant une quantité importante de méthane pour créer du gaz industriel, généralement appelé biogaz, afin d'offrir une alternative au gaz naturel. Ceci permet en outre de valoriser les déchets de type biomasse.

Usuellement, on a recours à des étapes de gazéification pour forcer la méthanisation des biomasses et en extraire du méthane.

De telles étapes ne permettent toutefois pas de récupérer des gaz très chargés en méthane. Les gaz en sortie comportent ainsi typiquement entre 2 et 6 % de méthane ce qui est très loin du taux de 95% du gaz naturel.

Le document US 2009/007484 propose un dispositif de production de matériaux carbonés et/ou hydrocarbonés à partir de biomasse, le dispositif comprenant à cet effet une enceinte chauffée et des moyens de convoyage de la biomasse dans l'enceinte. L'enceinte est par : ailleurs munie d'une sortie d'extraction du gaz issu de la décomposition de la biomasse dans l'enceinte, sortie raccordée à un condenseur.

### OBJET DE L'INVENTION

Un but de l'invention est de proposer un dispositif permettant de générer un gaz méthane ainsi que l'utilisation de ce dispositif pour la valorisation de produit type matière CSR ou matière polymérique.

### BREVE DESCRIPTION DE L'INVENTION

En vue de la réalisation de ce but, on propose un dispositif de production d'un gaz méthane par traitement thermique d'un produit sous forme de solides divisés, le dispositif comportant :
- une enceinte comprenant une entrée d'alimentation en produit, une sortie basse de récupérations des résidus du produit traité et une sortie haute d'extraction du gaz issu du traitement du produit,
- des moyens de convoyage du produit entre l'entrée de l'enceinte et la sortie basse de l'enceinte qui comprennent une vis montée pour tourner dans l'enceinte selon un axe géométrique de rotation et qui comprennent des moyens d'entraînement en rotation de la vis,
- des moyens de chauffage par effet Joule de la vis,
- une unité d'élimination d'impuretés présentes dans le gaz issu du traitement thermique du produit, ladite unité étant raccordée à la sortie haute de l'enceinte, et
- un système de purification du gaz en sortie de l'unité d'élimination, le système de purification étant raccordé à l'unité d'élimination.

De la sorte le produit est introduit à l'entrée de l'enceinte sous forme de solides divisés et la vis pousse de façon continue les solides divisés vers la sortie basse de l'enceinte. Du fait du mode de chauffage avantageux de la vis par effet Joule, les solides divisés se chauffent très rapidement et se transforment sans coller à la spire de la vis générant ainsi un gaz présentant déjà un fort taux en méthane (supérieur à 50% lorsque le produit est de type CSR (composés solides de récupération) ou matière polymérique tel que du plastique). L'association ultérieure de deux ensembles consécutifs de traitement du gaz permet dans un premier temps de débarrasser le gaz d'impuretés de types poussières, particules solides, goudrons, huiles ... puis dans un deuxième temps de purifier le gaz en séparant le méthane des autres composants gazeux.

On obtient ainsi en sortie de l'invention un gaz très riche en méthane. Des expérimentations effectuées par la demanderesse ont ainsi permis d'obtenir des taux de méthane sensiblement de 92%.

L'invention permet ainsi d'obtenir un gaz ayant une concentration en méthane proche de celle d'un gaz naturel. Le gaz obtenu par l'invention s'avère donc être une très bonne alternative au gaz naturel.

De plus, le gaz en sortie de l'invention s'avère directement injectable dans des contenants (bouteilles, citernes ...) ou dans un réseau de distribution de gaz.

En outre, l'invention peut être alimentée en produit de toute sorte comme des biomasses mais s'avère particulièrement avantageuse avec des produits de type matière CSR (composés solides de récupération) ou matière polymérique comme le plastique. Ceci s'avère particulièrement avantageux dans un cadre toujours plus important de valorisation des déchets, en particulier les déchets non fermentescibles pour lesquels les solutions de valorisation sont moins développées.

On distingue ainsi dans la présente invention, l'unité d'élimination d'impuretés qui sont indésirables et polluantes de façon générale du système de purification qui ne permet pas de nettoyer le gaz mais de l'enrichir en méthane en le séparant d'autres gaz.

Par « gaz méthane » on entend au sens de l'invention un gaz ayant comme composant majoritaire du méthane étant entendu que ledit gaz peut comprendre en plus faible proportions d'autres composants comme du diazote.

Selon un aspect particulier de l'invention, le dispositif comporte une cheminée d'entrée qui est connectée à l'entrée de l'enceinte et qui comprend des moyens de raccordement étanches à l'entrée de l'enceinte de sorte à limiter l'air entrant dans l'enceinte.

Selon un aspect particulier de l'invention, le dispositif comporte une cheminée de sortie qui est connectée à la sortie basse de l'enceinte et qui comprend des moyens de raccordement étanches à la sortie basse de l'enceinte de sorte à limiter l'air entrant dans l'enceinte.

Selon l'invention, l'unité d'élimination d'impuretés comporte des moyens de craquage du gaz.

Selon l'invention, l'unité d'élimination d'impuretés comporte des moyens de filtrage de poussières et de particules solides présentes dans le gaz.

Selon l'invention, les moyens de filtrage sont raccordés à la sortie des moyens de craquage.

Selon un aspect particulier de l'invention, les moyens de filtrage comportent un cyclone haute température ou un filtre céramique haute température.

Selon un aspect particulier de l'invention, le système de purification comporte au moins deux étages de purification distincts.

Selon un aspect particulier de l'invention, le système de purification comporte trois étages de purification.

Selon l'invention, le système de purification comporte des moyens de condensation des phases condensables du gaz à une pression inférieure à la pression atmosphérique prise au niveau de la mer.

Selon un aspect particulier de l'invention, les moyens de condensation sont conformés de sorte à assurer la séparation du gaz et des phases condensables à une pression comprise entre 0.04 et 0.3 bar.

Selon un aspect particulier de l'invention, le système de purification comporte un appareil d'adsorption par inversion de pression.

Selon un aspect particulier de l'invention, l'appareil d'adsorption par inversion de pression est raccordé à la sortie des moyens de condensation.

Selon un aspect particulier de l'invention, le système de purification comporte une machine de filtration.

Selon un aspect particulier de l'invention, la machine de filtration est une machine par séparation membranaire.

Selon un aspect particulier de l'invention, la machine de filtration est raccordée à la sortie de l'appareil d'absorption par inversion de poussée.

Par ailleurs, l'invention concerne l'utilisation du dispositif précité à la valorisation d'un produit de type matière CSR ou matière polymérique.

### BREVE DESCRIPTION DES DESSINS

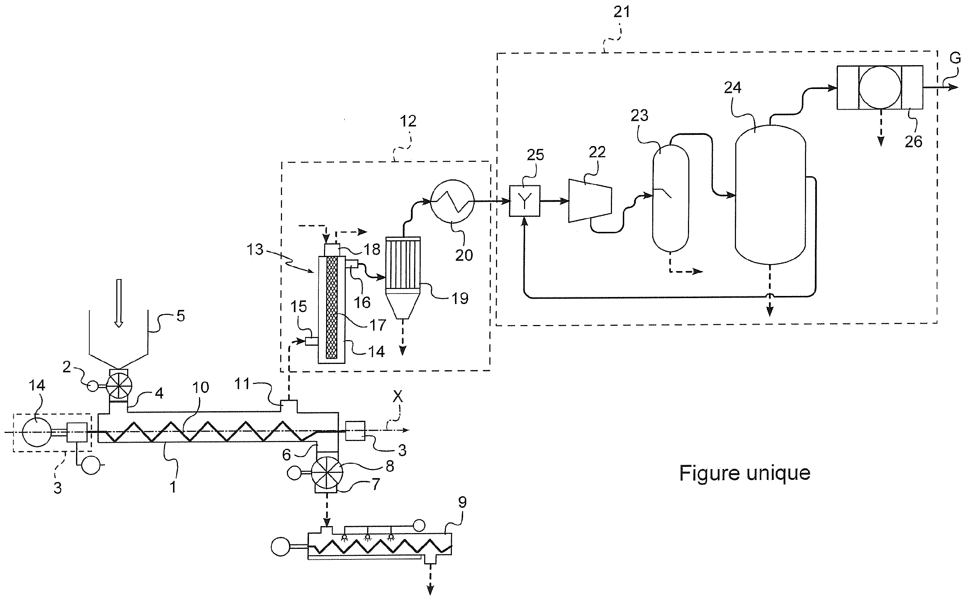

L'invention sera mieux comprise à la lumière de la description qui suit en référence à la figure unique schématisant le dispositif selon un mode de réalisation particulier non limitatif de l'invention.

### DESCRIPTION DETAILLEE DE L'INVENTION

En référence à la figure unique, le dispositif selon un mode de réalisation particulier de l'invention permet de produire un gaz méthane par traitement thermique, ici par pyrolyse, d'un produit sous forme de solides divisés.

Le produit est par exemple formé de matière polymérique. Le produit est typiquement du plastique comprenant majoritairement du polyéthylène et du polytéréphtalate d'éthylène. De façon particulière, les solides divisés se présentent sous la forme de granulés en trois dimensions de type granules ou pellets. Les dimensions maximales desdits solides divisés sont de préférence comprises entre 2 et 30 millimètres.

Le dispositif selon l'invention comporte une enceinte 1, de direction générale essentiellement horizontale, qui est maintenue à distance du sol par des piètements (non représentés ici). L'enceinte 1 comporte une enveloppe externe, ici unitaire, qui est par exemple métallique, en particulier réalisée en acier inox amagnétique. Selon un mode de réalisation particulier, l'enceinte 1 comporte en outre ici une enveloppe interne unitaire en matériau réfractaire. Un caisson technique 3 est fixé à chacune des extrémités de l'enceinte 1.

L'enceinte 1 comporte ici une entrée 4 d'alimentation en produit de l'enceinte 1, entrée 4 qui est aménagée dans le couvercle de l'enceinte 1 sensiblement au niveau d'une première extrémité de l'enceinte 1.

Bien entendu, le fond et le couvercle de l'enceinte 1 sont définis par rapport au sol sur lequel repose l'enceinte 1.

Selon un mode de réalisation particulier, le dispositif comporte une cheminée d'entrée 5 qui est connectée à l'entrée 4 de l'enceinte.

De préférence, la cheminée d'entrée 5 comporte des moyens de raccordement étanches 2 à l'entrée 4 de l'enceinte 1 de sorte à limiter l'air entrant dans l'enceinte 1, air qui réduirait le taux en méthane du gaz en sortie d'enceinte ce qui n'est pas souhaité. Ces moyens de raccordement étanches 2 permettent également de contrôler le débit du produit versé dans l'enceinte 1. Lesdits moyens de raccordement étanches 2 comportent par exemple un sas hermétique agencé entre la cheminée d'entrée 5 et l'entrée 4 de l'enceinte 1 et commandé par des vannes.

La cheminée d'entrée 5 est par exemple connectée à une trémie d'alimentation ou encore à une unité de broyage, de compactage ou de granulation du produit en solides divisés ou encore à une unité de préconditionnement des solides divisés, une unité de préconditionnement permettant de chauffer et/ou de sécher les solides divisés à des valeurs prescrites de température et d'humidité relative ou encore de densifier lesdits solides divisés.

L'enceinte 1 comporte en outre une sortie basse 6 aménagée ici dans le fond de l'enceinte 1 sensiblement au niveau de la deuxième des deux extrémités de l'enceinte 1. Selon un mode de réalisation particulier, le dispositif comporte une cheminée de sortie 7 qui est connectée à la sortie basse 6 de l'enceinte 1.

De préférence, la cheminée de sortie 7 comporte des moyens de raccordement étanches 8 à la sortie basse 6 de l'enceinte 1 de sorte à limiter l'air entrant dans l'enceinte 1, air qui réduirait le taux en méthane du gaz en sortie d'enceinte 1 ce qui n'est pas souhaité. Ces moyens de raccordement étanches 8 permettent également de contrôler le débit d'évacuation des résidus du produit traité thermiquement dans l'enceinte 1. Lesdits moyens de raccordement étanches 8 comportent par exemple un sas hermétique agencé entre la cheminée de sortie et la sortie basse et commandé par des vannes.

La cheminée de sortie 7 est par exemple connectée à une unité de refroidissement 9 des résidus soit dans un but de destruction des résidus soit dans un but de valorisation desdits résidus qui pourront par exemple être utilisés en tant que combustibles, moyennant éventuellement une ou plusieurs étapes de traitement supplémentaire.

Par ailleurs, le dispositif comprend des moyens de convoyage du produit entre l'entrée de l'enceinte et la sortie basse de l'enceinte. Lesdits moyens comportent ainsi une vis 10 qui s'étend ici dans l'enceinte 1 selon un axe géométrique X entre les deux caissons techniques 3 et qui est montée pour tourner autour dudit axe géométrique X dans l'enceinte 1. La vis 10 est par exemple en acier inoxydable réfractaire. La vis 10 est ainsi résistante à de hautes températures typiquement comprises entre 700 et 1200 degrés. La vis 10 a ici une forme de serpentin hélicoïdal qui est fixé, par exemple par soudure, à ses deux extrémités en bout d'un tronçon d'arbre. Chacun desdits tronçon d'arbre est relié à son autre extrémité, par le biais d'une bride, à un arbre coaxial qui traverse le caisson technique d'extrémité associé.

Les moyens de convoyage comportent en outre des moyens d'entraînement en rotation de la vis 10 autour de l'axe géométrique X qui sont ici agencés dans l'un des caissons techniques 3. Selon un aspect particulier de l'invention, les moyens d'entraînement en rotation comportent un moteur électrique 14 et des moyens de liaison mécanique entre l'arbre de sortie du moteur et une extrémité de l'arbre coaxial associé, l'arbre coaxial entraînant lui-même la vis 10. Les moyens d'entraînement en rotation comportent ici des moyens de contrôle de la vitesse de rotation de l'arbre de sortie du moteur qui comprennent par exemple un variateur de vitesses. Les moyens de contrôle permettent ainsi d'adapter la vitesse de rotation de la vis 10 au produit convoyé c'est-à-dire d'adapter le temps de séjour du produit dans l'enceinte 1.

Le dispositif comporte en outre des moyens de chauffage par effet Joule de la vis 10 qui sont ici agencés dans les caissons techniques 3. Selon un mode de réalisation particulier, les moyens de chauffage comportent des moyens de génération d'un courant électrique et des moyens de raccordement des deux extrémités de la vis aux deux polarités desdits moyens de génération. A cet effet, chaque arbre coaxial est rigidement solidaire d'un tambour coaxial en matériau électriquement conducteur, sur lequel frottent des charbons d'amenée du courant électrique, reliés par des fils conducteurs (non représentés ici) aux moyens de génération d'un courant électrique. La vis 10 est ainsi traversée par la même intensité tout le long de l'axe géométrique X.

De préférence, la vis 10 est conformée de sorte à avoir une résistance électrique variant le long de son axe X et permettant ainsi d'offrir simultanément des zones de chauffages différentes le long de son axe X. De façon particulière, la vis 10 est ainsi conformée de sorte à avoir un profil de température tel que la température en entrée 4 de l'enceinte 1 soit supérieure à la température en sortie 6, 11 de l'enceinte 1. Ceci permet de limiter le collage des solides divisés en matière plastique sur la spire de la vis 10 à leur entrée dans l'enceinte 1 du fait de la fonte desdits solides divisés sous l'action du chauffage de la vis 10.

Selon un aspect particulier de l'invention, les moyens de chauffage comportent des moyens de régulation de l'intensité du courant électrique traversant la vis 10. Les moyens de régulation comportent ici un gradateur interposé entre les moyens de génération du courant électrique et les moyens de raccordement. Les moyens de régulation permettent ainsi d'adapter l'intensité électrique traversant la vis 10 au produit convoyé.

L'enceinte 1, les moyens de convoyage et les moyens d'alimentation forment donc ainsi ici un réacteur de pyrolyse pour le produit introduit dans l'enceinte 1.

Par ailleurs, l'enceinte 1 comporte également une sortie haute 11 pour l'extraction des gaz issus de la pyrolyse du produit, ladite sortie haute 11 étant aménagée ici dans le couvercle de l'enceinte 1 sensiblement au niveau de la deuxième des deux extrémités de l'enceinte 1. La sortie haute 11 est ici légèrement en amont de la sortie basse de l'enceinte 1 relativement à l'entrée 4 de l'enceinte.

Le dispositif comporte en outre une unité d'élimination 12 d'impuretés présentes dans le gaz de la pyrolyse du produit. Ladite unité 12 est raccordée à la sortie haute 11 de sorte que le gaz soit extrait en continu de l'enceinte 1 (au contraire de l'entrée 4 et de la sortie basse 6 qui sont conformées de sorte que l'alimentation en produit et l'évacuation des résidus puissent se faire de façon discontinue).

Selon l'invention, l'unité d'élimination 12 d'impuretés comporte des moyens de craquage du gaz qui sont ici directement raccordés à la sortie haute 11 de l'enceinte 1.

Ces moyens de craquage vont permettre de craquer les goudrons et les phases huileuses présents dans le gaz de sorte à récupérer en sortie des moyens de craquage un gaz plus propre.

Les moyens de craquage comportent par exemple un four de craquage 13 comportant un bâti 14 tubulaire verticale qui comprend une entrée 15 pour l'introduction du gaz et une sortie 16 pour l'évacuation dudit gaz hors du bâti 14, des moyens de chauffage dudit gaz qui comprennent un tube de chauffage 17 s'étendant verticalement à l'intérieur du bâti et coaxialement au bâti, le tube de chauffage 17 étant conformé de sorte à avoir son extrémité inférieure fermée, et étant agencé de sorte que son extrémité inférieure soit agencée dans le bâti 14 et de sorte que son extrémité supérieure soit raccordée à un brûleur 18 des moyens de chauffage qui est agencé à l'extérieur du bâti 14. Les moyens de chauffage comportent typiquement une conduite d'entrée d'un combustible de chauffage (gaz naturel, fuel, gaz de synthèse épuré, ou encore gaz traité par le présent four de craquage 13 dont une partie est prélevée au niveau de la sortie 16 du four de craquage 13 pour alimenter la conduite d'entrée ...) raccordé au brûleur 18 desdits moyens de chauffage. Les moyens de chauffage comportent également une conduite de sortie du combustible brûlé.

Ainsi, l'agencement particulier du bâti 14 et du tube de chauffage 17 permet de créer une zone de traitement dans laquelle le gaz est bien confiné. Ceci permet de favoriser les échanges thermiques au sein du bâti 14 entre le gaz à traiter et le tube de chauffage 17 : le craquage des goudrons et phases huiles s'avère donc efficace et rapide de telle sorte que le gaz récupéré en sortie de four de craquage 13 présente une bonne pureté.

Le four de craquage 13 est ici agencé de sorte que le gaz soit chauffé à environ 1100 degrés Celsius pour améliorer encore davantage le craquage.

De préférence, les moyens de chauffage utilisent d'abord un combustible de chauffage extérieur au four de craquage 13 pour initialiser le chauffage du tube de chauffage 17 (de type gaz naturel, fuel, gaz de synthèse épuré ...) et une fois le traitement du gaz débuté, les moyens de chauffage prélèvent une partie du gaz traité en sortie 16 du four de craquage 13 pour assurer le chauffage du tube de chauffage 17.

De la sorte, le four de craquage 13 s'avère relativement autonome et ne requiert un combustible extérieur que pour initialiser le début du craquage.

Le combustible extérieur pourra également être utilisé en cours de fonctionnement, lorsque le simple prélèvement du gaz traité en sortie 16 du four de craquage 13 n'est pas suffisant pour alimenter le brûleur 18.

De préférence, l'entrée 15 s'étend sensiblement tangentiellement au bâti 14. L'entrée 15 est donc agencée de sorte à faire pénétrer le gaz dans le bâti 15 le long de la paroi interne du bâti 15. Ceci permet de générer un effet cyclonique de sorte que le gaz s'écoule hélicoïdalement dans le bâti 15. Ceci favorise le traitement du gaz.

L'unité d'élimination 12 comporte en outre ici également des moyens de filtrage 19 qui sont par exemple directement raccordés à la sortie du four de craquage 13 afin d'éliminer poussières et particules solides encore présentes dans le gaz. Les moyens de filtrage 19 comportent typiquement un cyclone haute température et/ou un filtre haute température (tel qu'un filtre céramique) agencé en travers de la canalisation raccordée à la sortie du four de craquage 13. Le cyclone et/ou le filtre est ainsi résistant à de hautes températures typiquement comprises entre 600 et 1000 degrés Celsius.

De préférence, l'unité d'élimination 12 comporte également des moyens de refroidissement 20 de type échangeur thermique directement raccordés aux moyens de filtrage 19. Ceci permet de refroidir le gaz qui a été chauffé au cours de l'étape de craquage avant que le gaz ne soit purifié. Le gaz en entrée des moyens de refroidissement est à une température comprise par exemple entre 600 et 800 degrés Celsius. On refroidit par exemple le gaz aux environs de 40 degrés.

Par ailleurs, le dispositif comporte un système de purification 21 du gaz en sortie de l'unité d'élimination 12. Le système de purification 21 est ici directement raccordé à la sortie des moyens de refroidissement 20.

Selon un aspect particulier de l'invention, le système de purification 21 comporte trois étages de purification du gaz.

De préférence, le premier étage comporte des moyens de condensation des phases condensables du gaz à une pression inférieure à la pression atmosphérique prise au niveau de la mer (qui est sensiblement de 1 bar). Typiquement les moyens de condensation sont conformés de sorte à assurer la séparation du gaz et des phases condensables à une pression comprise entre 0.04 et 0.3 bar et de préférence à une pression de 0.14 bar.

A cet effet, les moyens de condensation comportent un compresseur 22 comprimant le gaz à la pression visée, ici de 0.14 bar, compresseur par ailleurs raccordé à la sortie des moyens de refroidissement 20. Les moyens de condensation comportent en outre un condenseur 23 qui est lui-même directement raccordé à la sortie du compresseur 22 et qui va permettre de séparer le gaz des phases condensables à 0.14 bar. En pratique, la majorité de ces phases condensables est de l'eau. On augmente ainsi la teneur en méthane du gaz en sortie du premier étage.

De façon particulière, le deuxième étage comporte un appareil d'adsorption par inversion de pression 24 qui est directement raccordé à la sortie du premier étage.

Un tel appareil est bien connu de l'art antérieur et ne sera pas détaillé davantage ici.

Ceci va permettre de retirer quasiment en totalité non seulement le dioxyde de carbone mais également le diazote encore présents dans le gaz permettant ainsi d'augmenter encore davantage la teneur en méthane du gaz en sortie du deuxième étage.

De préférence, le premier étage et le deuxième étage sont interconnectés. A cet effet, le premier étage comporte un mélangeur 25 agencé en amont du compresseur 22 et directement connecté d'une part à la sortie des moyens de refroidissement 20 et d'autre part au compresseur 22. L'appareil d'adsorption par inversion de pression 24 est par ailleurs raccordé au mélangeur 25.

Le mélangeur 25 permet ainsi de mélanger le gaz en sortie de l'unité d'élimination 12 avec une partie du gaz en cours de purification par le deuxième étage pour favoriser ladite purification et ainsi transmettre au troisième étage un gaz plus pur en méthane.

De façon particulière, le troisième étage comporte une machine de filtration 26 qui est ici de type machine par séparation membranaire. Une telle machine 26 est bien connue de l'art antérieur et ne sera pas détaillée davantage ici.

Ladite machine 26 est ici directement raccordée à la sortie du deuxième étage.

Ceci va permettre de retirer quasiment en totalité le dihydrogène encore présents dans le gaz permettant ainsi d'augmenter encore davantage la teneur en méthane du gaz en sortie du troisième étage (noté G) et donc en sortie du système de purification.

Une mise en œuvre du dispositif va être à présente décrite.

Tout d'abord, le produit à traiter est introduit dans la cheminée d'entrée 5 sous forme de solides divisés et la vis 10 pousse de façon continue les solides divisés vers la sortie basse 6 de l'enceinte 1. Du fait de la température de la vis 10, les solides divisés se ramollissent progressivement jusqu'à fondre ce qui va générer du gaz déjà chargé en méthane.

La vis 10 assure ainsi à la fois un traitement thermique du produit et le convoyage du produit.

De préférence, le traitement thermique du produit se fait à haute température dans l'enceinte 1, typiquement entre 500 et 1000 degrés Celsius et préférentiellement entre 600 et 800 degrés Celsius.

De préférence, le dispositif est conformé de sorte que le produit demeure entre 10 et 30 minutes dans l'enceinte et encore plus préférentiellement entre 15 et 20 minutes.

Ceci permet donc de pyrolyser efficacement le produit et ainsi de récupérer en sortie haute 11 de l'enceinte 1 un gaz déjà très chargé en méthane. Typiquement le gaz en sortie de l'enceinte 1 présente un taux en méthane supérieur à 60%.

Les résidus carbonés du produit sont alors évacués au niveau de la sortie basse 6.

Par ailleurs, le gaz extrait en sortie haute 11 de l'enceinte 1 traverse l'unité d'élimination 12 des impuretés qui va permettre de retirer successivement goudrons et huiles puis poussières et particules. On récupère ainsi un gaz plus propre en sortie de ladite unité d'élimination 12.

Puis le gaz traverse le système de purification 21 qui va permettre de retirer à son tour successivement eau, dioxyde de carbone, diazote et dihydrogène. On récupère ainsi en sortie du dispositif un gaz G plus pur en ce qui concerne le taux de méthane.

Le gaz G en sortie du système de purification, et donc du dispositif, s'avère avoir ainsi un très fort taux en méthane. Typiquement le gaz G en sortie du dispositif présente un taux en méthane supérieur à 90%.

Bien entendu l'invention n'est pas limitée au mode de réalisation décrit et on peut y apporter des variantes de réalisation sans sortir du cadre de l'invention tel que défini par les revendications.

En particulier, bien qu'ici le produit alimentant le dispositif soit du plastique comprenant majoritairement du polyéthylène et du polytéréphtalate d'éthylène, le dispositif pourra utiliser d'autre type de produit pour la production de méthane. Le produit pourra ainsi aussi bien être par exemple une biomasse qu'un solide polymérique, comme un déchet de plastique, de caoutchouc ou d'élastomère ou un encore un solide comprenant du carton, une matière métallique tel que de l'aluminium ... ou encore un combustible solide de récupération. On rappelle qu'une biomasse désigne les fractions biodégradables des produits, déchets et résidus provenant de l'industrie en général et de l'agriculture, de la sylviculture et des industries connexes en particulier.

Le produit pourra comprendre un seul type de solide (polymérique, plastique, CSR, biomasse ...) ou plusieurs types de solide. Les solides divisés pourront se présenter sous la forme de granulés en trois dimensions ou encore de feuillets en deux dimensions. De façon générale, les solides divisés pourront se présenter sous forme de poudre, de granulés, de morceaux, de fibres

Par ailleurs, l'enceinte et les moyens de convoyage et de chauffage par effet joule associés pourront être différents de ce qui a été indiqué. Par exemple, les moyens de raccordement étanches de l'entrée d'alimentation et/ou de la sortie basse pourront comprendre d'autres éléments qu'un sas comme par exemple une vanne écluse ou encore un appareil de dosage. La vis et les moyens de chauffage par effet joule associés pourront ainsi être conformés pour autoriser un chauffage par pallier du produit, la vis présentant par exemple une résistance électrique variant le long de son axe et permettant ainsi d'offrir simultanément des zones de chauffages différentes le long de son axe.

Le dispositif pourra être conformé de sorte que l'enceinte soit remplie d'un gaz inerte pour limiter ou pour éliminer la présence d'oxygène dans l'enceinte.

## Revendications

1. Dispositif de production d'un gaz méthane (G) par traitement thermique d'un produit sous forme de solides divisés, le dispositif comportant :
- une enceinte (1) comprenant une entrée (4) d'alimentation en produit, une sortie basse (6) de récupérations des résidus du produit traité et une sortie haute (11) d'extraction du gaz issu du traitement du produit,
- des moyens de convoyage du produit entre l'entrée de l'enceinte et la sortie basse de l'enceinte qui comprennent une vis (10) montée pour tourner dans l'enceinte selon un axe géométrique de rotation (X) et qui comprennent des moyens d'entraînement en rotation de la vis,
- des moyens de chauffage par effet Joule de la vis,
- une unité d'élimination (12) d'impuretés présentes dans le gaz issu du traitement thermique du produit, ladite unité étant raccordée à la sortie haute de l'enceinte et comportant des moyens de craquage du gaz (13) et des moyens de filtrage (19) de poussières et de particules solides présentes dans le gaz, les moyens de filtrage (19) étant raccordés à la sortie des moyens de craquage, et
- un système de purification (21) du gaz en sortie de l'unité d'élimination, le système de purification étant raccordé à l'unité d'élimination, le système de purification (21) comportant des moyens de condensation (23) des phases condensables du gaz à une pression inférieure à la pression atmosphérique prise au niveau de la mer.

2. Dispositif selon la revendication 1, comportant une cheminée d'entrée (5) qui est connectée à l'entrée (4) de l'enceinte et qui comprend des moyens de raccordement étanches (2) à l'entrée de l'enceinte de sorte à limiter l'air entrant dans l'enceinte.

3. Dispositif selon la revendication 1 ou la revendication 2, comportant une cheminée de sortie (7) qui est connectée à la sortie basse de l'enceinte et qui comprend des moyens de raccordement étanches (8) à la sortie basse (6) de l'enceinte de sorte à limiter l'air entrant dans l'enceinte.

4. Dispositif selon la revendication 1, dans lequel les moyens de filtrage (19) comportent un cyclone haute température ou un filtre céramique haute température.

5. Dispositif selon l'une des revendications précédentes, le système de purification comporte au moins deux étages de purification distincts.

6. Dispositif selon la revendication précédente, dans lequel le système de purification comporte trois étages de purification.

7. Dispositif selon la revendication 1, dans lequel les moyens de condensation (23) sont conformés de sorte à assurer la séparation du gaz et des phases condensables à une pression comprise entre 0.04 et 0.3 bar.

8. Dispositif selon l'une des revendications précédentes, dans lequel le système de purification (21) comporte un appareil d'adsorption par inversion de pression (24).

9. Dispositif selon la revendication 8, dans lequel l'appareil d'adsorption par inversion de pression (24) est raccordé à la sortie des moyens de condensation (23) .

10. Dispositif selon l'une des revendications précédentes, dans lequel le système de purification (21) comporte une machine de filtration (26).

11. Dispositif selon la revendication précédente, dans lequel la machine de filtration (26) est une machine par séparation membranaire.

12. Dispositif selon l'une des revendications 8 à 9 et selon l'une des revendications 10 à 11, dans lequel la machine de filtration (26) est raccordée à la sortie de l'appareil d'absorption par inversion de poussée (24).

13. Application du dispositif selon l'une des revendications précédentes, dans lequel le produit alimentant l'enceinte (1) est de type matière CSR ou matière polymérique.

14. Procédé de production d'un gaz méthane (G) par traitement thermique d'un produit sous forme de solides divisés à l'aide d'un dispositif selon l'une des revendications 1 à 12, dans lequel on alimente l'enceinte (1) en produits en matière CSR ou en matière polymérique.

## Patentansprüche

1. Vorrichtung zum Herstellen von Methangas (G) durch Wärmebehandlung eines Produkts in Form von geteilten Feststoffen, wobei die Vorrichtung umfasst:
- eine Einfassung (1), die einen Produktzuführungseinlass (4), einen unteren Auslass (6) zur Rückgewinnung von Rückständen des behandelten Produkts und einen oberen Auslass (11) zur Extraktion des aus der Behandlung des Produktes stammenden Gases umfasst,
- Fördermittel zum Fördern des Produktes zwischen dem Einlass der Einfassung und dem unteren Auslass der Einfassung, wobei die Fördermittel eine Schnecke (10), die in der Einfassung um eine geometrische Rotationsachse (X) drehbar gelagert ist, und Antriebsmittel zum Drehantrieb der Schnecke umfassen,
- Heizmittel zum Heizen der Schnecke mittels Joule-Effekt,
- eine Eliminierungseinheit (12) zum Eliminieren von Verunreinigungen, die in dem aus der Wärmebehandlung des Produktes stammenden Gas vorhanden sind, wobei die Einheit mit dem oberen Auslass der Einfassung verbunden ist und Krackmittel (13) zum Kracken des Gases und Filtermittel (19) zum Filtern von Staub und in dem Gas vorhandenen Feststoffpartikeln umfasst, wobei die Filtermittel (19) an den Auslass der Krackmittel angeschlossen sind, und
- ein Reinigungssystem (21) zum Reinigen des Gases am Auslass der Eliminierungseinheit, wobei das Reinigungssystem an die Eliminierungseinheit angeschlossen ist, wobei das Reinigungssystem (21) Kondensationsmittel (23) zum Kondensieren kondensierbarer Phasen des Gases bei einem Druck umfasst, der geringer als der Atmosphärendruck ist, der auf Meereshöhe gemessen wird.

2. Vorrichtung nach Anspruch 1, umfassend einen Einlasskamin (5), der mit dem Einlass (4) der Einfassung verbunden ist und der dichte Verbindungsmittel (2) zur dichten Verbindung mit dem Einlass der Einfassung umfasst, sodass die in die Einfassung eintretende Luft begrenzt ist.

3. Vorrichtung nach Anspruch 1 oder Anspruch 2, umfassend einen Auslasskamin (7), der mit dem unteren Auslass der Einfassung verbunden ist und der dichte Verbindungsmittel (8) zur dichten Verbindung mit dem unteren Auslass (6) der Einfassung umfasst, sodass die in die Einfassung eintretende Luft begrenzt ist.

4. Vorrichtung nach Anspruch 1, bei der die Filtermittel (19) einen Hochtemperatur-Zyklon oder ein Hochtemperatur-Keramikfilter umfassen.

5. Vorrichtung nach einem der vorhergehenden Ansprüche, bei der das Reinigungssystem mindestens zwei unterschiedliche Reinigungsstufen umfasst.

6. Vorrichtung nach dem vorhergehenden Anspruch, bei der das Reinigungssystem drei Reinigungsstufen umfasst.

7. Vorrichtung nach Anspruch 1, bei der die Kondensationsmittel (23) so angepasst sind, dass sie die Trennung von Gas und kondensierbaren Phasen bei einem Druck zwischen 0,04 und 0,3 bar sicherstellen.

8. Vorrichtung nach einem der vorhergehenden Ansprüche, bei der das Reinigungssystem (21) eine Druckwechsel-Adsorptionsvorrichtung (24) umfasst.

9. Vorrichtung nach Anspruch 8, bei der die Druckwechsel-Adsorptionsvorrichtung (24) an den Auslass der Kondensationsmittel (23) angeschlossen ist.

10. Vorrichtung nach einem der vorhergehenden Ansprüche, bei der das Reinigungssystem (21) eine Filtermaschine (26) umfasst.

11. Vorrichtung nach dem vorhergehenden Anspruch, bei der die Filtermaschine (26) eine Membrantrennmaschine ist.

12. Vorrichtung nach einem der Ansprüche 8 bis 9 und nach einem der Ansprüche 10 bis 11, bei der die Filtermaschine (26) an den Auslass der Druckwechsel-Adsorptionsvorrichtung (24) angeschlossen ist.

13. Nutzung der Vorrichtung nach einem der vorhergehenden Ansprüche, bei der das der Einfassung (1) zugeführte Produkt vom Typ EBS-Material oder Polymermaterial ist.

14. Verfahren zum Herstellen eines Methangases (G) durch Wärmebehandlung eines Produktes in Form von geteilten Feststoffen mittels einer Vorrichtung nach einem der Ansprüche 1 bis 12, bei dem man der Einfassung (1) Produkte aus EBS-Material oder Polymermaterial zuführt.

## Claims

1. Device for producing a methane gas (G) by heat-treating a substance in the form of divided solids, the device comprising:
• an enclosure (1) comprising a substance feed inlet (4), a bottom outlet (6) for recovering residues of the treated substance, and a top outlet (11) for extracting the gas coming from the treatment of the substance;
• conveyor means for conveying the substance between an inlet of the enclosure and the bottom outlet, the means comprising a screw (10) mounted to rotate inside the enclosure about an axis of rotation (X) and having drive means for driving the screw in rotation;
• heater means for heating the screw by the Joule effect;
• an impurity elimination unit (12) for eliminating impurities present in the gas coming from the heat treatment substance, said unit being connected to the top outlet of the enclosure and comprising cracking means (13) for cracking the gas and filter means (19) for filtering dust and solid particles present in the gas, filter means (19) being connected to the outlet of the cracking means; and
• a purification system (21) for purifying the gas at the outlet of the elimination unit, the purification system being connected to the elimination unit, the purification system (21) comprising condensation means (23) for condensing condensable phases of the gas at a pressure that is less than atmospheric pressure measured at sea level.

2. Device according to claim 1, including an inlet chimney (5) that is connected to the inlet (4) of the enclosure and that comprises leaktight connection means (2) for connection to the inlet of the enclosure so as to limit the air entering the enclosure.

3. Device according to claim 1 or claim 2, including an outlet chimney (7) that is connected to the bottom outlet (4) of the enclosure and that comprises leaktight connection means (8) for connection to the bottom outlet of the enclosure so as to limit the air entering the enclosure.

4. Device according to claim 1, wherein the filter means (19) comprise a high-temperature cyclone or a high-temperature ceramic filter.

5. Device according to any preceding claim, the purification system comprises at least two distinct purification stages.

6. Device according to the preceding claim, wherein the purification system comprises at least three purification stages.

7. Device according to claim 1, wherein the condensation means (23) are shaped so as to ensure separation of the gas from the condensable phases at a pressure lying in the range 0.04 bar to 0.3 bar.

8. Device according to any preceding claim, wherein the purification system (21) comprises pressure swing adsorption equipment (24).

9. Device according to claim 8, wherein the pressure swing adsorption equipment (24) is connected to the outlet of the condensation means (23).

10. Device according to any preceding claim, wherein the purification system (21) comprises a filtering machine (26).

11. Device according to the preceding claim, wherein the filtering machine (26) is a membrane separation machine.

12. Device according to claim 8 or claim 9 and claims 10 to 11, wherein the filtering machine (26) is connected to the outlet of the pressure swing adsorption equipment (24).

13. Application of the device according to any preceding claim, wherein the substance feeding the enclosure (1) is of the SRF type or of the polymer material type.

14. Method of producing a methane gas (G) by heat-treating a substance in the form of divided solids by means of a device according to any one of claims 1 to 12, wherein the enclosure (1) is supplied with substances made of SRF or polymer material.
